# FASCICULE DE BREVET EUROPEEN

(11) **EP 2 341 964 B1**
(45) Date de publication et mention de la délivrance du brevet: **18.01.2017**
(21) Numéro de dépôt: 09794037.3
(22) Date de dépôt: 10.07.2009
(51) Int. Cl.: A61M 15/00

(54) **DISPOSITIF D'INHALATION DE POUDRE**
PULVERINHALATIONSVORRICHTUNG
POWDER INHALATION DEVICE

(30) Priorité: 11.07.2008 FR 0854756
(43) Date de publication de la demande: 13.07.2011
(73) Titulaire: Aptar France SAS, 27110 Le Neubourg (FR)
(72) Inventeur: FAGOT, Christophe, 78250 Mezy (FR)
(74) Mandataire: CAPRI
(86) Numéro de dépôt international: PCT/FR2009/051374
(87) Numéro de publication internationale: WO 2010/004223

(56) Documents cités:
- WO-A-2005/025550
- FR-A- 2 881 118
- FR-A- 2 909 641
- GB-A- 2 179 260
- GB-A- 2 375 310
- US-A1- 2001 027 790

## Description

La présente invention concerne un dispositif d'inhalation de poudre, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a changé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation.

Pour assurer une distribution finement pulvérisée de la poudre, le document US 6 715 486 décrit une chambre de dispersion contenant une ou plusieurs billes entraînées en rotation par l'écoulement d'air et de poudre se dirigeant du réservoir ouvert vers l'orifice de distribution. Cette chambre de dispersion assure une bonne désaglomération de la poudre, et a un effet positif sur la résistance à l'écoulement en la diminuant. Les effets de cette chambre à bille sont toutefois relativement sensibles à l'orientation de l'inhalateur au moment de l'inhalation, avec des propriétés de rendement, de variabilité ou de résistance qui pourront être affectés en cas d'orientation non optimale, correspondant à l'inhalateur maintenu autrement que verticalement. Les documents FR-2 909 641, WO 2005/025550 et GB-2 375 310 décrivent d'autres dispositifs de l'état de la technique.

La présente invention a pour but de fournir un dispositif d'inhalation de poudre, en particulier un inhalateur de poudre sèche qui ne reproduit pas les inconvénients susmentionnés.

En particulier, la présente invention a pour but de fournir un tel inhalateur qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a aussi pour but de fournir un tel inhalateur qui assure une bonne précision de dosage et une bonne reproductibilité de dosage à chaque actionnement, indépendamment de l'orientation de l'inhalateur.

La présente invention a donc pour objet un dispositif d'inhalation de poudre, comportant un corps pourvu d'un orifice de distribution, au moins un réservoir contenant une dose de poudre à distribuer, des moyens d'ouverture de réservoir pour ouvrir un réservoir à chaque actionnement, une chambre de dispersion comportant une sortie reliée audit orifice de distribution et une entrée reliée auxdits moyens d'ouverture et recevant la dose de poudre à partir dudit réservoir ouvert, ladite chambre de dispersion contenant au moins une bille, ladite chambre de dispersion comportant un chemin de bille dont la largeur diminue à partir de ladite entrée dans le sens de déplacement de ladite au moins une bille.

Avantageusement, le bord externe dudit chemin de bille est circulaire ou elliptique.

Avantageusement, la largeur maximale dudit chemin de bille est inférieure au double du diamètre d'une bille.

Avantageusement, ledit chemin de bille comporte une surface de fond sensiblement plane, un bord externe sensiblement circulaire et un bord interne sensiblement circulaire formé par un profil central, l'axe central dudit bord externe étant décalé de l'axe central dudit bord interne.

Avantageusement, ladite chambre de dispersion comporte une partie de fond et une partie de couvercle.

Avantageusement, ledit profil central est formé sur la partie de fond.

Avantageusement, ladite entrée est tangentielle dans ladite chambre de dispersion.

Avantageusement, ladite chambre de dispersion contient une pluralité de billes, notamment six.

Avantageusement, toutes les billes sont de mêmes dimensions.

Avantageusement, lesdits moyens d'ouverture sont des moyens de perçage comportant une aiguille adaptée à percer un réservoir à chaque actionnement.

Avantageusement, lesdits moyens d'ouverture sont commandés par l'inhalation de l'utilisateur, de sorte que le réservoir est simultanément ouvert et vidé, la poudre entraînée par l'écoulement d'inhalation traversant ladite chambre de dispersion avant d'être expulsée à travers l'orifice de distribution.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique en section transversale d'un inhalateur de poudre,
- la figure 2 représente une vue schématique en section transversale de côté d'une partie du dispositif d'inhalation de la figure 1, selon un mode de réalisation avantageux de l'invention,
- la figure 3 est une vue similaire à celle de la figure 2, en section transversale de dessus, et
- la figure 4 est une schématique en perspective éclatée de la partie du dispositif d'inhalation des figures 2 et 3.

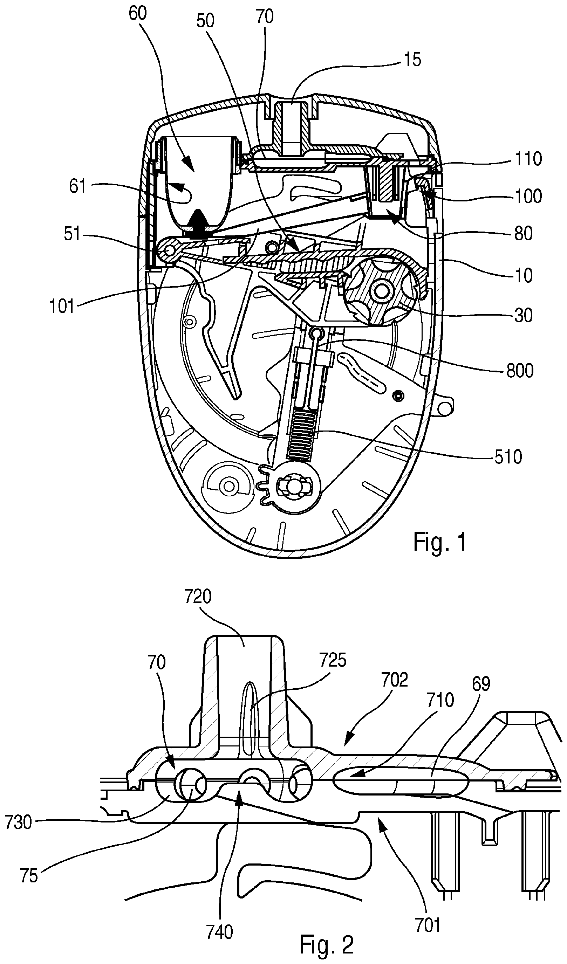

Sur la figure 1 est représentée une variante de réalisation avantageuse d'un inhalateur de poudre sèche. Cet inhalateur comporte un corps 10 sur lequel peuvent être montées coulissantes ou pivotantes deux parties formant un capot (non représentées) adaptées à être ouvertes pour ouvrir et charger le dispositif. Le corps 10 peut être de forme environ arrondie, mais il pourrait avoir tout autre forme appropriée. Le corps 10 comporte un embout buccal ou d'inhalation définissant un orifice de distribution 15 à travers lequel l'utilisateur va inhaler lors de l'actionnement du dispositif. Les capots peuvent s'ouvrir par pivotement autour d'un axe de rotation commun, mais tout autre moyen d'ouverture du dispositif est envisageable. En variante, le dispositif pourrait comporter un seul capot au lieu de deux.

A l'intérieur du corps 10, il est prévu une bande (non représentée) de réservoirs individuels, également appelés blisters, réalisée sous forme d'une bande souple allongée sur laquelle les blisters sont disposés les uns derrière les autres, de manière connue. Avant la première utilisation, la bande de blister peut être enroulée à l'intérieur du corps 10, de préférence dans une partie de stockage, et des premiers moyens de déplacement de bande 30 sont prévus pour progressivement dérouler et faire avancer cette bande de blister. Des seconds moyens de déplacement 50, 51 sont prévus pour amener un réservoir individuel ou blister respectif dans une position de distribution à chaque actionnement du dispositif. La partie de bande comportant les réservoirs vides est avantageusement adaptée à s'enrouler dans un autre endroit dudit corps 10, de préférence une partie de réception.

L'inhalateur comporte des moyens d'ouverture de réservoir 80 (qui ne sont indiqués sur la figure 1 que de manière très schématique) comportant de préférence des moyens de perçage et/ou de coupage de la couche de fermeture des blisters. Par exemple, les moyens d'ouverture de réservoir comportent avantageusement une aiguille, de préférence fixe par rapport au corps 10, et contre laquelle un blister respectif est déplacé à chaque actionnement par les seconds moyens de déplacement. Le blister est alors percé par ladite aiguille, qui pénètre dans ledit blister pour expulser la poudre au moyen du flux d'inhalation de l'utilisateur.

Les premiers moyens de déplacement sont adaptés à faire avancer la bande de blisters avant et/ou pendant et/ou après chaque actionnement du dispositif. Les seconds moyens de déplacement sont adaptés à déplacer le réservoir à vider contre lesdits moyens de perçage et/ou de coupage lors de l'actionnement. Ces seconds moyens de déplacement peuvent être sollicités, via des moyens de chargement 800, par un élément élastique 510, tel qu'un ressort ou tout autre élément élastique équivalent, ledit élément élastique pouvant être préchargé lors de l'ouverture du dispositif. De préférence, les premiers moyens de déplacement comportent une roue d'indexage 30 qui reçoit et guide les blisters. Une rotation de cette roue d'indexage fait avancer la bande de blister. Dans une position angulaire particulière, un réservoir donné est toujours en position face aux moyens d'ouverture. Les seconds moyens de déplacement peuvent comporter un élément de support 50 rotatif autour d'un axe de rotation 51, ladite roue d'indexage 30 étant montée rotative sur ledit élément de support.

Un cycle d'actionnement du dispositif peut être le suivant. Lors de l'ouverture du dispositif, les deux parties latérales formant capot sont écartées l'une de l'autre en pivotant sur le corps pour ouvrir le dispositif, et ainsi charger le dispositif. Dans cette position la roue d'indexage ne peut pas se déplacer vers l'aiguille car les seconds moyens de déplacement sont retenus par des moyens de blocage 100, 110 appropriés. De préférence, c'est lors de l'inhalation par l'utilisateur à travers l'embout buccal que ces moyens de blocage sont débloqués, ce qui provoque alors le pivotement dudit élément de support 50 et donc le déplacement de ladite roue d'indexage 30 en direction de l'aiguille,et donc l'ouverture d'un réservoir.

L'orientation optimale de l'inhalateur lors de son utilisation correspond à une position sensiblement verticale avec l'orifice distribution 15 dirigé vers le haut, comme représenté sur la figure 1.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation, qui comporte avantageusement une unité 60 déplaçable et/ou déformable sous l'effet de l'inhalation, cette unité étant adaptée à libérer les moyens de blocage 100, 110, par exemple via une tige 101. Cette unité comprend avantageusement une chambre d'air déformable 61. L'inhalation de l'utilisateur provoque la déformation de ladite chambre d'air déformable, permettant ainsi de libérer lesdits moyens de blocage et donc de permettre le déplacement des seconds moyens de déplacement, et donc d'un réservoir respectif vers sa position d'ouverture. Le réservoir n'est donc ouvert qu'au moment de l'inhalation, de sorte qu'il est simultanément vidé. Il n'y a donc aucun risque de perte de dose entre l'ouverture du réservoir et son vidage.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

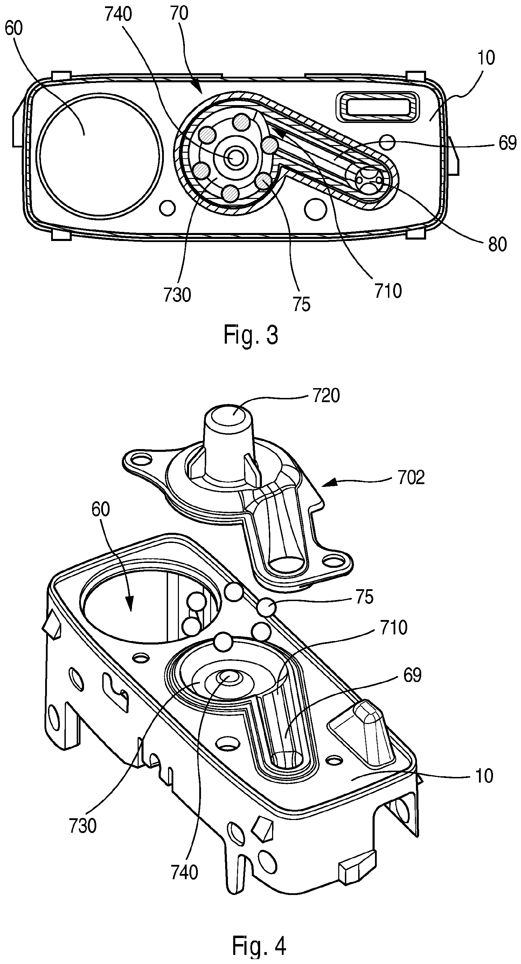

L'inhalateur comporte en outre une chambre de dispersion 70 qui est destinée à recevoir la dose de poudre après ouverture d'un réservoir respectif 21. Cette chambre de dispersion 70 est pourvue d'au moins une bille 75, de préférence six billes comme visible sur les figures 3 et 4, qui se déplacent à l'intérieur de ladite chambre 70 pendant l'inhalation, pour améliorer la distribution du mélange air et poudre après ouverture d'un réservoir, afin d'augmenter l'efficacité du dispositif.

Cette chambre de dispersion 70 est de préférence de forme circulaire ou elliptique, avec une entrée 710 de préférence tangentielle dans ladite chambre et une sortie 720 perpendiculaire, de préférence orientée selon un axe vertical passant environ au centre de ladite chambre de dispersion 70. De préférence, la chambre de dispersion 70 est formée de deux parties, une partie de fond 701 et une partie de couvercle 702 assemblées l'une sur l'autre au moment de l'assemblage du dispositif. Avantageusement, la sortie 720 est formée sur la partie de couvercle 702, alors que l'entrée 710 est formée par les deux parties, à savoir la partie de fond 701 et la partie de couvercle 702. La chambre de dispersion 70 comporte un chemin de bille 730, qui suit de préférence environ la forme de celle-ci, à savoir un cercle ou une ellipse selon les cas. Ce chemin de bille 730 comporte avantageusement une surface de fond sensiblement plane et deux parois de bord latérales courbées ou incurvées pour permettre un déplacement rapide des billes. De préférence, ce chemin de bille 730 est défini radialement à l'intérieur par une projection centrale 740, qui est réalisée de préférence sur la partie de fond 701. Ceci est avantageux notamment pour l'assemblage, les billes 75 venant automatiquement se placer dans le chemin de bille 730 avant la fixation de la partie de couvercle 702 sur la partie de fond 701. En variante, un profil approprié pourrait être prévu sur la partie de couvercle, comme indiqué sur la figure 1. Le profil central 740 est de préférence disposé face à la sortie 720 de la chambre de dispersion 70, comme visible sur la figure 2. La sortie 720 comporte de préférence une ou plusieurs restrictions 725 à l'intérieur du canal pour empêcher une expulsion de la ou des billes 75 prévue(s) dans la chambre de dispersion 70. Ceci est une sécurité dans l'hypothèse où une bille 75 devait s'échapper du chemin de bille, par exemple lors de l'assemblage. Dans le mode réalisation préféré, la chambre de dispersion 70 comporte plusieurs billes 75, de préférence six, et ces billes ont de préférence les mêmes dimensions. Pour permettre le déplacement rapide des billes 75 le long du chemin de bille 730, ce chemin de bille a une largeur qui est supérieure au diamètre des billes (ou supérieure au diamètre de la bille la plus grande si les billes ont des dimensions différentes). On peut prévoir des chemins de billes 730 suffisamment larges pour permettre à deux billes d'être disposées côte à côte dans ledit chemin de bille, mais de préférence, le chemin de bille 730 est conçu pour ne permettre le passage que d'une bille à la fois. Dans ce mode de réalisation avantageux, la largeur du chemin de bille 730 est donc inférieure au double du diamètre des billes. Comme visible sur les figures 3 et 4, l'entrée 710 relie la chambre de dispersion 70 à l'élément de perçage 80 via un canal 69. Dans les variantes représentées, les billes 75 tournent dans le sens inverse des aiguilles d'une montre, mais il est entendu que le canal 69 qui mène à l'entrée de la chambre de dispersion pourrait être disposé dans une autre orientation avec les billes 75 tournant dans le sens des aiguilles d'une montre à l'intérieur de la chambre de dispersion. De même, l'entrée 710 n'est pas nécessairement parfaitement tangentielle, et selon les cas, il pourrait même être souhaitable de prévoir une entrée 710 légèrement désaxée par rapport à la tangente.

Selon l'invention, le chemin de bille 730 a une largeur décroissante dans le sens de déplacement des billes en partant de l'entrée 710 de la chambre de dispersion. Le chemin de bille 730 a alors une forme dite en « escargot » ou en « spirale » qui se rétrécit au fur et à mesure qu'on s'éloigne de ladite entrée d'air 710. Ceci a pour effet d'accélérer le flux d'air dans ledit chemin de bille 730 et donc de diminuer les rétentions de poudre au niveau des parois de bord de ladite chambre de dispersion, en particulier dans les zones éloignées de l'entrée 710, dans le sens de l'écoulement. Dans l'exemple représenté sur les figures 3 et 4, le sens de rotation est inverse au sens des aiguilles d'une montre, mais l'entrée d'air 710 pourrait être orientée différemment avec un sens de rotation inversé c'est-à-dire dans le sens des aiguilles d'une montre pour les billes. Dans cette seconde hypothèse, le rétrécissement de la largeur serait bien entendu également inversé par rapport à celui représenté sur les figures 3 et 4.

Avantageusement, la largeur décroissante du chemin de bille 730 est réalisée en désaxant le profil ou projection central 740 par rapport à l'axe de symétrie de la chambre de dispersion 70. La surface de fond sensiblement plane du chemin de bille 730 se rétrécit ainsi comme visible sur les figures 3 et 4. Ainsi, la surface de fond plane du chemin de bille 730 se rétrécit, le bord externe étant circulaire, le bord interne étant circulaire, et l'axe central dudit bord externe étant décalé de l'axe central dudit bord interne. Comme visible sur les figures 2 et 4, ces bords interne et externe sont incurvés pour former un chemin de bille arrondi.

Après l'inhalation, lorsque l'utilisateur referme le dispositif, tous les composants reviennent vers leur position de repos initiale. Le dispositif est alors prêt pour un nouveau cycle d'utilisation.

L'inhalateur de poudre sèche tel que décrit précédemment procure notamment les fonctions suivantes :
▪ une pluralité de doses individuelles de poudre stockées dans des réservoirs individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération préchargé ;
▪ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
▪ Une dispersion efficace de la poudre avant son expulsion, pour limiter les rétentions de poudre et pour garantir une bonne précision et reproductibilité de dosage à chaque actionnement, même en cas d'orientation non optimale de l'inhalation.

D'autres fonctions sont également fournies par le dispositif de l'invention tel que cela a été décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, indépendamment de la forme de la chambre de dispersion, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées.

## Revendications

1. Dispositif d'inhalation de poudre, comportant un corps (10) pourvu d'un orifice de distribution (15), au moins un réservoir contenant une dose de poudre à distribuer, des moyens d'ouverture de réservoir (80) pour ouvrir un réservoir à chaque actionnement, une chambre de dispersion (70) comportant une sortie (720) reliée audit orifice de distribution (15) et une entrée (710) reliée auxdits moyens d'ouverture et recevant la dose de poudre à partir dudit réservoir ouvert, ladite chambre de dispersion (70) contenant au moins une bille (75), **caractérisé en ce que** ladite chambre de dispersion (70) comporte un chemin de bille (730) dont la largeur diminue à partir de ladite entrée (710) dans le sens de déplacement de ladite au moins une bille (75).

2. Dispositif selon la revendication 1, dans lequel le bord externe dudit chemin de bille (730) est circulaire ou elliptique.

3. Dispositif selon les revendications 1 ou 2, dans lequel la largeur maximale dudit chemin de bille (730) est inférieure au double du diamètre de ladite au moins une bille (75).

4. Dispositif selon l'une quelconque des revendications précédentes, dans lequel le chemin de bille (730) comporte une surface de fond sensiblement plane, un bord externe sensiblement circulaire et un bord interne sensiblement circulaire formé par un profil central (740), l'axe central dudit bord externe étant décalé de l'axe central dudit bord interne.

5. Dispositif selon l'une quelconque des revendications précédentes, dans lequel ladite chambre de dispersion (70) comporte une partie de fond (701) et une partie de couvercle (702).

6. Dispositif selon les revendications 4 et 5, dans lequel ledit profil central (740) est formé sur la partie de fond (701).

7. Dispositif selon l'une quelconque des revendications précédentes, dans lequel ladite entrée (710) est tangentielle dans ladite chambre de dispersion (70).

8. Dispositif selon l'une quelconque des revendications précédentes, dans lequel ladite chambre de dispersion (70) contient une pluralité de billes (75), notamment six.

9. Dispositif selon la revendication 8, dans lequel toutes les billes (75) sont de mêmes dimensions.

10. Dispositif selon l'une quelconque des revendications précédentes, dans lequel lesdits moyens d'ouverture sont des moyens de perçage comportant une aiguille adaptée à percer un réservoir à chaque actionnement.

11. Dispositif selon l'une quelconque des revendications précédentes, dans lequel lesdits moyens d'ouverture sont commandés par l'inhalation de l'utilisateur, de sorte que le réservoir est simultanément ouvert et vidé, la poudre entraînée par l'écoulement d'inhalation traversant ladite chambre de dispersion (70) avant d'être expulsée à travers l'orifice de distribution (15).

## Patentansprüche

1. Vorrichtung zum Inhalieren eines Pulvers, die einen mit einer Abgabeöffnung (15) versehenen Körper (10), wenigstens einen eine Dosis des abzugebenden Pulvers enthaltenden Behälter, Behälter-Öffnungseinrichtungen (80), die dazu dienen, bei jeder Betätigung einen Behälter zu öffnen, eine Dispersionskammer (70) die einen mit der Abgabeöffnung (15) verbundenen Ausgang (720) und einen mit den Öffnungseinrichtungen verbundenen Eingang (710) umfasst und die Pulverdosis aus dem offenen Behälter empfängt, wobei diese Dispersionskammer (70) wenigstens eine Kugel (75) enthält, **dadurch gekennzeichnet, dass** die Dispersionskammer (70) einen Kugelweg (730) umfasst, dessen Weite ausgehend von dem besagten Eingang (710) in Richtung der Bewegung der wenigstens einen Kugel (75) abnimmt.

2. Vorrichtung nach Anspruch 1, bei welcher der äußere Rand des Kugelweges (730) kreisförmig oder elliptisch ist.

3. Vorrichtung nach Anspruch 1 oder 2, bei welcher die maximale Größe des Kugelweges (730) kleiner ist als der doppelte Durchmesser der wenigstens einen Kugel (75).

4. Vorrichtung nach einem der vorhergehenden Ansprüche, bei welcher der Kugelweg (730) eine im Wesentlichen ebene Bodenoberfläche, einen im Wesentlichen kreisförmigen Außenrand und einen im Wesentlichen kreisförmigen Innenrand umfasst, der von einem zentralen Profil (740) gebildet wird, wobei die zentrale Achse des Außenrandes gegen die zentrale Achse des Innenrandes versetzt ist.

5. Vorrichtung nach einem der vorhergehenden Ansprüche, bei welcher die Dispersionskammer (70) einen Bodenteil (701) und einen Deckelteil (702) aufweist.

6. Vorrichtung nach den Ansprüchen 4 und 5, bei welcher das zentrale Profil (740) auf dem Bodenteil (701) ausgebildet ist.

7. Vorrichtung nach einem der vorhergehenden Ansprüche, bei welcher der Eingang (710) tangential in die Dispersionskammer (70) verläuft.

8. Vorrichtung nach einem der vorhergehenden Ansprüche, bei welcher die Dispersionskammer (70) eine Vielzahl von Kugeln (75), insbesondere sechs Kugeln enthält.

9. Vorrichtung nach Anspruch 8, bei welcher alle Kugeln (75) die gleichen Abmessungen aufweisen.

10. Vorrichtung nach einem der vorhergehenden Ansprüche, bei welcher die Öffnungseinrichtungen Durchbohrungseinrichtungen sind, die eine Nadel umfassen, die geeignet ist, bei jeder Betätigung einen Behälter zu durchbohren.

11. Vorrichtung nach einem der vorhergehenden Ansprüche, bei welcher die Öffnungseinrichtungen durch das Einatmen des Verwenders derart gesteuert werden, dass der Behälter gleichzeitig geöffnet und entleert wird, wobei das Pulver durch die Inhalationsströmung mitgenommen wird, die durch die Dispersionskammer (70) hindurch erfolgt, bevor es durch die Abgabeöffnung (15) hindurch ausgestoßen wird.

## Claims

1. A powder inhaler comprising: a body (10) that is provided with a dispenser orifice (15); at least one reservoir containing a dose of powder for dispensing; reservoir-opening means (80) for opening a reservoir on each actuation; and a dispersion chamber (70) including an outlet (720) that is connected to said dispenser orifice (15), and an inlet (710) that is connected to said opening means and that receives the dose of powder from said open reservoir, said dispersion chamber (70) containing at least one ball (75), **characterized in that** said dispersion chamber (70) includes a ball path (730) having a width that decreases from said inlet (710) in the direction of displacement of said at least one ball (75).

2. A device according to claim 1, wherein the outer edge of said ball path (730) is circular or elliptical.

3. A device according to claim 1 or claim 2, wherein the maximum width of said ball path (730) is less than twice the diameter of said at least one ball (75).

4. A device according to any preceding claim, wherein the ball path (730) comprises a bottom surface that is substantially plane, an outer edge that is substantially circular, and an inner edge that is substantially circular and that is formed by a central profile (740), the central axis of said outer edge being offset from the central axis of said inner edge.

5. A device according to any preceding claim, wherein said dispersion chamber (70) comprises a base portion (701) and a cover portion (702).

6. A device according to claim 4 and claim 5, wherein said central profile (740) is formed on the base portion (701).

7. A device according to any preceding claim, wherein said inlet (710) is tangential in said dispersion chamber (70).

8. A device according to any preceding claim, wherein said dispersion chamber (70) contains a plurality of balls (75), in particular six.

9. A device according to claim 8, wherein all of the balls (75) have the same dimensions.

10. A device according to any preceding claim, wherein said opening means are perforator means comprising a needle that is adapted to perforate a reservoir on each actuation.

11. A device according to any preceding claim, wherein said opening means are controlled by the user inhaling, such that the reservoir is opened and emptied simultaneously, the powder driven by the inhalation flow passing through said dispersion chamber (70) prior to being expelled through the dispenser orifice (15).
